# EUROPEAN PATENT SPECIFICATION

(11) **EP 4 130 741 B1**
(45) Date of publication and mention of the grant of the patent: **28.01.2026**
(21) Application number: 21782113.1
(22) Date of filing: 30.03.2021
(51) Int. Cl.: G01N 33/53, G01N 33/531, G01N 33/543

(54) **METHOD FOR IMMUNOASSAY OF AMYLOID BETA IN BLOOD, AND KIT FOR SAME**
VERFAHREN ZUM IMMUNTEST VON AMYLOID BLUT UND KIT DAFÜR
PROCÉDÉ DE DOSAGE IMMUNOLOGIQUE DU BETA-AMYLOÏDE DANS LE SANG ET KIT ASSOCIÉ

(30) Priority: 31.03.2020 JP 2020063156
(43) Date of publication of application: 08.02.2023
(73) Proprietor: Fujirebio Inc., Tokyo 107-0052 (JP)
(72) Inventor: GU, Ran, Tokyo 1070052 (JP)
(74) Representative: Mewburn Ellis LLP
(86) International application number: PCT/JP2021/013528
(87) International publication number: WO 2021/200940

(56) References cited:
- EP-A1- 3 373 005
- EP-B2- 1 478 931
- WO-A1-2015/052654
- JP-A- 2005 519 088
- JP-A- 2006 507 488
- JP-A- 2011 081 011
- JP-A- 2018 151 162
- JP-A- H0 658 935
- US-A- 5 164 295
- US-A1- 2019 145 965
- US-B2- 9 766 216
- PESINI PEDRO ET AL: "Reliable Measurements of the [beta] -Amyloid Pool in Blood Could Help in the Early Diagnosis of AD", vol. 2012, 16 August 2012 (2012-08-16), pages 1 - 10, XP093140551, ISSN: 2090-8024, Retrieved from the Internet <URL:http://downloads.hindawi.com/journals/ijad/2012/604141.pdf> DOI: 10.1155/2012/604141
- ERIKSSON SUSANN ET AL: "Negative Interference in Cardiac Troponin I Immunoassays from a Frequently Occurring Serum and Plasma Component", CLINICAL CHEMISTRY, vol. 49, no. 7, 1 July 2003 (2003-07-01), US, pages 1095 - 1104, XP093299828, ISSN: 0009-9147, DOI: 10.1373/49.7.1095
- TIMMER, N. M ET AL.: "Aggregation and cytotoxic properties towards cultured cerebrovascular cells of Dutch -mutated Abeta40 (DAbeta1-40) are modulated by sulfate moieties of heparin", NEUROSCIENCE RESEARCH, vol. 66, 30 December 2009 (2009-12-30), pages 380 - 389, XP026923632, DOI: 10.1016/j.neures.2009.12.012

## Description

### TECHNICAL FIELD

The present invention relates to a method of immunoassay of blood amyloid β, and a kit therefor.

### BACKGROUND ART

Amyloid β is known as a biomarker for Alzheimer's disease. Measurement of amyloid β in a sample by an immunoassay is known (Patent Document 1), and kits for the measurement are commercially available. Most of the commercially available immunoassay kits for amyloid β use a cerebrospinal fluid (CSF) as the sample. On the other hand, patients with Alzheimer's disease are known to have amyloid β also in blood. Since collection of CSF is invasive, it would be advantageous if amyloid β in a blood sample can be immunologically assayed.
Patent Document 2 discloses methods for selectively capturing aggregated PrP protein in the presence of the non-aggregated form of the protein.
Non-Patent Document 1 discloses that commercial assay kits can be used to take Aβ measurements in undiluted plasma, diluted plasma and cellular fractions for diagnosis of AD.
Patent Document 3 relates to reagents and methods for measuring cardiac troponin I.
Patent Document 4 relates to migration shift assays.
Patent Document 5 relates to immunoassays employing sulfated polysaccharides in particular for measuring soluble interleukin-2 receptor and prostate specific antigen.

### PRIOR ART DOCUMENT

### [Patent Document]

[Patent Document 1] JP 2006-091025A
[Patent Document 2] EP 1 478 931 B2
[Patent Document 3] EP 3 373 005 A1
[Patent Document 4] US 9 766 216 B2
[Patent Document 5] WO 2017/179611

### [Non-Patent Document]

[Non-Patent Document 1] Pesini Pedro et al: "Reliable Measurements of the [beta] - Amyloid Pool in Blood Could Help in the Early Diagnosis of AD", INTERNATIONAL JOURNAL OF ALZHEIMER'S DISEASE, vol. 2012, 16 August 2012 (2012-08-16), pages 1-10

### SUMMARY OF THE INVENTION

### PROBLEMS TO BE SOLVED BY THE INVENTION

It had been thought that an immunoassay of amyloid β may be similarly possible for both CSF and blood since the same substance, amyloid β, is targeted in the measurement irrespective of whether the amyloid β is in CSF or blood. It was found, however, that an immunoassay of amyloid β in a blood sample results in a sensitivity lower than that in the case where CSF is used as a sample, which is problematic.

Accordingly, an object of the present invention is to provide an immunoassay method capable of highly sensitively measuring amyloid β in a blood sample, and a kit therefor.

### MEANS FOR SOLVING THE PROBLEMS

As a result of intensive study on the measurement of amyloid β in blood, the present inventors discovered that highly sensitive measurement of amyloid β in a blood sample is possible by carrying out antigen-antibody reaction between amyloid β in a blood sample and an anti-amyloid β antibody or an antigen-binding fragment thereof in the presence of an anionic polymer, thereby reaching the present invention.

That is, the present invention provides the following.
(1) A method of immunoassay of amyloid β in a blood sample, the method comprising carrying out the immunoassay in the presence of an anionic polymer.
(2) The method according to (1), wherein the anionic polymer is an anionic polymer comprising a side chain containing a sulfate group or a sulfo group.
(3) The method according to (2), wherein the anionic polymer is at least one selected from the group consisting of dextran sulfate and salts thereof; polystyrene sulfonic acid and salts thereof; and heparin sulfate and salts thereof.
(4) The method according to (3), wherein the anionic polymer is dextran sulfate sodium.
(5) The method according to any one of (1) to (4), wherein the immunoassay is a sandwich method, the method comprising bringing, in the presence of the anionic polymer, the amyloid β in the blood sample into contact with an anti-amyloid β antibody or an antigen-binding fragment thereof immobilized on a solid phase, to measure the amyloid β.
(6) The method according to any one of (1) to (5), wherein the amyloid β is amyloid β1-40 or amyloid β1-42.
(7) A kit for immunoassay of amyloid β in a blood sample, the kit comprising: an anti-amyloid β antibody or an antigen-binding fragment thereof; and an anionic polymer.
(8) The kit according to (7), wherein the immunoassay is a sandwich method, the kit comprising: a solid phase on which an anti-amyloid β antibody or an antigen-binding fragment thereof is immobilized; and an anionic polymer.
(9) The kit according to (8), wherein the solid phase is particles, the kit comprising a particle liquid containing the particles and the anionic polymer.

### EFFECT OF THE INVENTION

By the method of the present invention, amyloid β in a blood sample can be highly sensitively measured.

### BRIEF DESCRIPTION OF THE DRAWINGS

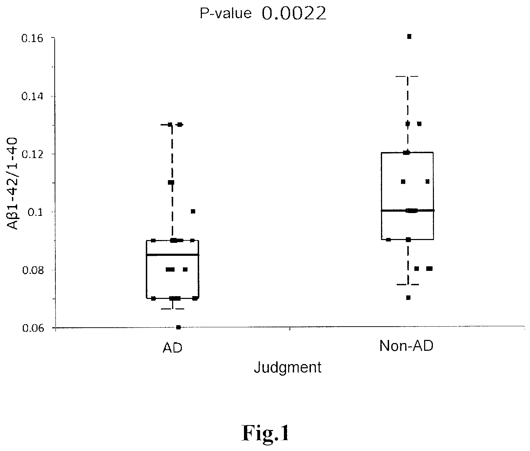

Fig. 1 is a diagram showing the Aβ₁₋₄₂/Aβ₁₋₄₀ ratios in patients with Alzheimer's disease and healthy individuals as measured in the Examples below.

### MODE FOR CARRYING OUT THE INVENTION

The sample to be subjected to the immunoassay method of the present invention is a blood sample. Examples of the blood sample include all of whole blood, serum, and plasma. The blood sample is preferably serum or plasma.

As concretely described in the Examples below, an experiment in which purified amyloid β is added to plasma and a buffer followed by performing an immunoassay to compare the recovery level (count value) of the amyloid β added was carried out to discover the presence of a substance (covering substance) that suppresses reaction between amyloid β and an anti-amyloid β antibody, which substance is eluted into a fraction corresponding to the molecular weight of 300 to 400 kDa in gel filtration chromatography. Further, as a result of a study, it was discovered that the binding suppression due to the covering substance can be reduced by carrying out the antigen-antibody reaction of the immunoassay in the presence of an anionic polymer. The present invention is based on such discoveries, and the most characteristic feature of the present invention is that the immunoassay is carried out in the presence of an anionic polymer.

The anionic polymer is preferably an anionic polymer comprising a side chain containing a sulfate group, such as dextran sulfate, heparin sulfate, chondroitin A sulfate, chondroitin B sulfate, chondroitin C sulfate, or a salt thereof; an anionic polymer comprising a side chain containing a sulfo group, such as polystyrene sulfonic acid or a salt thereof; or an anionic polymer comprising a carboxyl group, such as poly(meth)acrylic acid or a salt thereof. Among these, an anionic polymer comprising a side chain containing a sulfate group or a sulfo group is preferred. Dextran sulfate, polystyrene sulfonic acid, heparin sulfate, or a salt thereof is more preferred. Dextran sulfate, polystyrene sulfonic acid, or a salt thereof is especially preferred. In particular, dextran sulfate or a salt thereof is preferred. Examples of the salt include alkali metal salts such as sodium salt and potassium salt. These anionic polymers may be used individually, or in combination of two or more thereof.

The anionic polymer has an average molecular weight of usually about 1,000 to 5,000,000, about 2,000 to 4,000,000, about 3,000 to 2,000,000, about 4,000 to 1,000,000, preferably about 5,000 to 700,000, more preferably about 5,000 to 50,000 in terms of the mass average molecular weight.

It is clear that anionic polymers are effective for any immunoassay method since anionic polymers reduce the suppression of antigen-antibody reaction between amyloid β and an anti-amyloid β antibody or an antigen-binding fragment thereof (the term "antibody" hereinafter means "antibody or antigen-binding fragment thereof" unless the context clearly dictates otherwise), which suppression is caused by the covering substance. More specifically, immunoassays include the sandwich method, the competition method, the agglutination method, and the immunochromatography method, and the present invention includes any of these immunoassays. These immunoassay methods *per se* are well known, and do not need to be described herein in detail. A brief description, however, of each immunoassay is given below.

The sandwich method includes various methods such as chemiluminescent enzyme immunoassay (CLEIA), Enzyme-Linked ImmunoSorbent Assay (ELISA), radioimmunoassay, ElectroChemiluminescence Immunoassay (ECLIA), and Fluorescence Immunoassay (FIA). The present invention includes any of these methods. The immunoassay of the present invention is not limited, and may be the sandwich method. Examples of the sandwich method include the one-step sandwich method and the two-step sandwich method.

In the two-step sandwich method, for example, first, an anti-amyloid β antibody immobilized on a solid phase or an anti-amyloid β antibody to be immobilized on a solid phase (immobilization antibody) is brought into contact with amyloid β in a blood sample, to allow antigen-antibody reaction between the immobilization antibody and the amyloid β (primary reaction). In cases where the immobilization antibody is an anti-amyloid β antibody to be immobilized on a solid phase, the immobilization antibody is immobilized on the solid phase after the primary reaction. Thereafter, B/F separation is carried out. Subsequently, the amyloid β bound to the immobilization antibody is brought into contact with an anti-amyloid β antibody to which a labeling substance for detection is bound (labeled antibody), to allow antigen-antibody reaction between the amyloid β and the labeled antibody (secondary reaction). Subsequently, B/F separation is carried out, and then a signal derived from the label of the labeled antibody bound to the amyloid β bound to the solid phase is detected to enable measurement of amyloid β in the blood sample. After the B/F separation, washing may be carried out.

**In** the one-step sandwich method, an immobilization antibody, amyloid β in a sample, and a labeled antibody are brought into contact with each other to carry out, in a single step, antigen-antibody reaction between the immobilization antibody and the amyloid β, and antigen-antibody reaction between the amyloid β and the labeled antibody. In cases where the immobilized antibody is an anti-amyloid β antibody to be immobilized on a solid phase, the immobilization antibody is immobilized on the solid phase after the antigen-antibody reaction or at the same time as the antigen-antibody reaction. Subsequently, B/F separation is carried out, and then a signal derived from the label of the labeled antibody bound to the amyloid β bound to the solid phase is detected to enable measurement of amyloid β in the blood sample.

The direct competitive method is a method in which an antibody against a target antigen to be measured (in the present invention, amyloid β) is immobilized on a solid phase (immobilization), and blocking treatment (treatment of the solid phase with a solution of a protein such as serum albumin) for prevention of non-specific adsorption is carried out, followed by reacting this antibody with a test sample containing the target antigen and with a certain amount of labeled antigen, performing washing, and then quantifying the label bound to the solid phase. Since the antigen in the test sample and the labeled antigen competitively bind to the antibody, as the amount of the antigen in the test sample increases, the amount of the label bound to the solid phase decreases. Antigen standard solutions with various known concentrations are prepared, and the amount of the label (the absorbance, luminescence intensity, fluorescence intensity, or the like depending on the properties of the label; the same applies hereinafter) immobilized on the solid phase is measured for each solution. Thereafter, a calibration curve is prepared by plotting the antigen concentration along the abscissa, and the amount of the label along the ordinate. By measuring the amount of the label for an unknown test sample, and applying the measured amount of the label to the calibration curve, the amount of the antigen in the unknown test sample can be measured. The direct competitive method *per se* is well known in the art, and described in, for example, US 20150166678 A.

In the indirect competitive method, a target antigen (in the present invention, amyloid β) is immobilized. Subsequently, blocking treatment of the solid phase is carried out, and then a test sample containing the target antigen is mixed with a certain amount of an anti-target-antigen antibody, followed by reacting the resulting mixture with the immobilized antigen. After washing, the anti-target-antigen antibody bound to the solid phase is quantified. This can be carried out by reacting a labeled secondary antibody against the anti-target-antigen antibody, performing washing, and then measuring the amount of the label. Antigen standard solutions with various known concentrations are prepared, and the amount of the label immobilized on the solid phase is measured for each solution, followed by preparation of a calibration curve. By measuring the amount of the label for an unknown test sample, and applying the measured amount of the label to the calibration curve, the amount of the antigen in the unknown test sample can be measured. It is also possible to use a labeled primary antibody without using the labeled secondary antibody. The indirect competitive method *per se* is well known in the art, and described in, for example, the above-mentioned US 20150166678 A.

Among the immunoassays described above, chemiluminescent enzyme immunoassay (CLEIA), chemiluminescence immunoassay (CLIA), enzyme immunoassay (EIA), radioimmunoassay (RIA), and fluorescence immunoassay (FIA) are immunoassays classified based on the type of the label to be used when the direct competitive method, indirect competitive method, sandwich method, or the like described above is carried out. Chemiluminescent enzyme immunoassay (CLEIA) is an immunoassay which uses an enzyme (for example, alkaline phosphatase) as the label, together with a substrate (for example, AMPPD) that generates a chemiluminescent compound. Enzyme immunoassay (EIA) is an immunoassay which uses an enzyme (for example, peroxidase, alkaline phosphatase, luciferase, or β-galactosidase) as the label. As the substrate of each enzyme, a compound quantifiable by measurement of the absorbance or the like is used. For example, in the case of peroxidase, 1,2-phenylenediamine (OPD), 3,3',5,5'-tetramethylbenzidine (TMB), or the like is used. In the case of alkaline phosphatase, p-nitrophenyl phosphate (pNPP) or the like is used. In the case of β-galactosidase, MG: 4-methylumbelliferyl galactoside, NG: nitrophenyl galactoside, or the like is used. In the case of luciferase, luciferin or the like is used. Radioimmunoassay (RIA) is a method which uses a radioactive substance as the label. Examples of the radioactive substance include radioactive elements such as ³H, ¹⁴C, ³²P, ³⁵S, and ¹²⁵I. Fluorescence immunoassay (FIA) is a method which uses a fluorescent substance or a fluorescent protein as the label. Examples of the fluorescent substance or the fluorescent protein include fluorescein, fluorescein isothiocyanate, rhodamine, green fluorescent protein, and red fluorescent protein. Immunoassays *per se* using these labels are well known in the art, and described in, for example, US 8039223 B and US 20150309016 A1.

Turbidimetric immunoassay (TIA) is an immunoassay which utilizes the phenomenon that an antigen-antibody complex produced by binding between a target antigen to be measured (in the present invention, amyloid β) and an antibody against this antigen causes an increase in the turbidity. The antigen is added, at various known concentrations, to an anti-target-antigen antibody solution, and the turbidity of each resulting mixture is measured to prepare a calibration curve. By similarly measuring the turbidity of an unknown test sample, and applying the measured turbidity to the calibration curve, the amount of the antigen in the unknown test sample can be measured. Turbidimetric immunoassay *per se* is well known, and described in, for example, US 20140186238 A1. Latex agglutination is a method similar to turbidimetric immunoassay, but uses a suspension of latex particles whose surfaces have an anti-target-antigen antibody immobilized thereon, instead of the antibody solution in turbidimetric immunoassay. Turbidimetric immunoassay and latex agglutination *per se* are well known in the art, and described in, for example, US 7,820,398 B.

Immunochromatography is a method in which the above-described sandwich method or competitive method is carried out on a substrate (also called a matrix or a strip) formed of a porous material such as filter paper, cellulose membrane, glass fiber, or non-woven fabric. For example, in cases of immunochromatography by the sandwich method, a detection zone on which an anti-target-antigen antibody is immobilized is provided on the substrate, and a test sample containing a target antigen is added to the substrate, followed by allowing a developer to flow from the upstream side. This allows the target antigen to migrate to the detection zone, where the target antigen is immobilized on the detection zone. The immobilized target antigen is sandwiched with a labeled secondary antibody, and the label immobilized on the detection zone is detected to detect the target antigen in the test sample. By forming a label zone containing the labeled secondary antibody in the upstream side of the detection zone, the conjugate of the target antigen and the labeled secondary antibody can be immobilized on the detection zone. In cases where the label is an enzyme, a substrate zone containing a substrate of the enzyme is also provided in the upstream side of the detection zone. In cases of the competitive method, for example, the target antigen may be preliminarily immobilized on the detection zone, and the target antigen in the test sample may be allowed to compete with the target antigen immobilized on the detection zone. By providing a labeled antibody zone in the upstream side of the detection zone, allowing the target antigen in the test sample to react with the labeled antibody, immobilizing unreacted labeled antibody on the detection zone, and then detecting or quantifying the label, the target antigen in the test sample can be detected or quantified. Immunochromatography *per se* is well known in the art, and described in, for example, US 6210898 B.

Among the immunoassays described above, the sandwich method is preferred from the viewpoint of the detection sensitivity and the simplicity of automation. The sandwich method is especially preferably chemiluminescent enzyme immunoassay (CLEIA), which is an immunoassay using a magnetic particle as the solid phase, an enzyme (for example, alkaline phosphatase) as the label, and a substrate (for example, 3-(2'-spiroadamantane)-4-methoxy-4-(3'-phosphoryloxy)phenyl-1,2-dioxetane disodium salt (AMPPD)) that generates a chemiluminescent compound as the substrate.

The anionic polymer is made to be present in the reaction system in which the amyloid β in the blood sample is brought into contact with the anti-amyloid β antibody to carry out the antigen-antibody reaction between the amyloid β in the blood sample and the anti-amyloid β antibody.

For example, in the two-step sandwich method, the anionic polymer is preferably present in the reaction system in which the immobilization antibody is brought into contact with amyloid β in the sample to carry out the antigen-antibody reaction between the immobilization antibody and the amyloid β. In the one-step sandwich method, the anionic polymer is preferably present in the reaction system in which the immobilization antibody, amyloid β in the sample, and the labeled antibody are brought into contact with each other to carry out the antigen-antibody reaction.

The solid phase is not limited, and a solid phase used in a known immunoassay system may be used. Specific examples of the material of the solid phase include, but are not limited to, polystyrene, polyethylene, Sepharose, latex, dextran, agarose, gelatin, and polyacrylamide. The solid phase used is preferably a solid phase which easily allows immobilization of an antibody on its surface, and which easily allows separation of the immune complex formed during the assay from unreacted components. The solid phase is especially preferably a plastic plate, latex particle, or magnetic particle used in ordinary immunoassays. From the viewpoint of ease of handling, storage, ease of separation, and the like, a magnetic particle composed of the above-described material is most preferably used. Immobilization of the antibody to such a solid phase can be carried out by a conventional method well known to those skilled in the art. The immobilization of the antibody to the solid phase may be carried out by physical adsorption, or by the use of covalent bonding. The immobilization of the antibody to the solid phase may also be carried out by immobilizing one of affinity substances such as biotin-streptavidin to the solid phase, binding the other to the antibody, and then mixing these together to immobilize the antibody to the solid phase through the affinity substances.

The labeling substance is also not limited, and labeling substances used in known immunoassays may be similarly used. Specific examples of the labeling substance include enzymes, fluorescent substances, chemiluminescent substances, dyes, and radioactive substances. Examples of the enzymes that may be used include, but are not limited to, known enzymes such as alkaline phosphatase (ALP), peroxidase, and β-galactosidase.

Amyloid β is produced by cleavage from its precursor by an enzyme. Different cleavage enzymes produce different types of amyloid β, such as amyloid β composed of the amino acids at positions 1 to 42 (amyloid β1-42 (Aβ₁₋₄₂)), amyloid β composed of the amino acids at positions 1 to 40 (amyloid β1-40 (Aβ₁₋₄₀)), amyloid β composed of the amino acids at positions 5 to 42 (amyloid β5-42 (Aβ₅₋₄₂)), amyloid β composed of the amino acids at positions 5 to 40 (amyloid β5-40 (Aβ₅₋₄₀)), amyloid β composed of the amino acids at positions 11 to 42 (amyloid β11-42 (Aβ₁₁₋₄₂)), amyloid β composed of the amino acids at positions 11 to 40 (amyloid β11-40 (Aβ₁₁₋₄₀)), amyloid β composed of the amino acids at positions 16 to 42 (amyloid β16-42 (Aβ₁₆₋₄₂)), amyloid β composed of the amino acids at positions 16 to 40 (amyloid β16-40 (Aβ₁₆₋₄₀)), amyloid β composed of the amino acids at positions 17 to 42 (amyloid β17-42 (Aβ₁₇₋₄₂)), amyloid β composed of the amino acids at positions 17 to 40 (amyloid β17-40 (Aβ₁₇₋₄₀)), amyloid β composed of the amino acids at positions 20 to 42 (amyloid β20-42 (Aβ₂₀₋₄₂)), amyloid β composed of the amino acids at positions 20 to 40 (amyloid β20-40 (Aβ₂₀₋₄₀)), amyloid β composed of the amino acids at positions 21 to 42 (amyloid β21-42 (Aβ₂₁₋₄₂)), amyloid β composed of the amino acids at positions 21 to 40 (amyloid β21-40 (Aβ₂₁₋₄₀)), amyloid β composed of the amino acids at positions 35 to 42 (which is called amyloid β35-42 (Aβ₃₅₋₄₂)), and amyloid β composed of the amino acids at positions 35 to 40 (amyloid β35-40 (Aβ₃₅₋₄₀)). Aβ₁₋₄₂, Aβ₅₋₄₂, Aβ₁₁₋₄₂, Aβ₁₆₋₄₂, Aβ₁₇₋₄₂, Aβ₂₀₋₄₂, Aβ₂₁₋₄₂, and Aβ₃₅₋₄₂ are collectively referred to as amyloid βX-42. Aβ₁₋₄₀, Aβ₅₋₄₀, Aβ₁₁₋₄₀, Aβ₁₆₋₄₀, Aβ₁₇₋₄₀, Aβ₂₀₋₄₀, Aβ₂₁₋₄₀, and Aβ₃₅₋₄₀ are collectively referred to as amyloid βX-40. The present invention is useful for measurement of any type of amyloid β, and the scope of the present invention encompasses the measurement of any of these. The present invention is especially preferably used for measurement of amyloid β1-42 and amyloid β1-40.

Any anti-amyloid β antibody or the antigen-binding fragment thereof may be used as long as the antibody or the antigen-binding fragment thereof specifically binds to amyloid β,. The antibody may be either a monoclonal antibody or a polyclonal antibody. A monoclonal antibody is generally preferably used. Since methods of preparation of polyclonal antibodies and monoclonal antibodies are well known, the anti-amyloid β antibody may be prepared using an amyloid β antigen as an immunogen. The "antigen-binding fragment" may be any antibody fragment as long as the fragment retains the binding capacity (antigen-antibody reactivity) to the corresponding antigen of the original antibody. Specific examples of the antigen-binding fragment include, but are not limited to, Fab, F(ab')2, and scFv. As is well known, Fab and F(ab')2 can be obtained by treating an antibody with a protease such as papain or pepsin. A method of preparation of scFv (single chain fragment of variable region; single-chain antibody) is also well known, and scFv can be prepared according to the well-known method.

The anti-amyloid β antibody or the antigen-binding fragment thereof may be appropriately selected depending on the type of the amyloid β to be measured. Antibodies and antibody combinations therefor are known, and commercially available products may be used.

For example, in cases where amyloid β1-42 is to be measured by the sandwich method, an antibody (anti-Aβ42 antibody) that specifically binds to the C-terminal region of amyloid βX-42 and an antibody (anti-Aβ N-terminus antibody) that specifically binds to the N-terminal region including the first amino acid of amyloid β may be used. As described above, such an antibody may be either a commercially available antibody or an antibody produced by a well-known method. An anti-Aβ42 antibody may be used as the immobilization antibody, and an anti-Aβ N-terminus antibody may be used as the labeled antibody. Alternatively, an anti-Aβ N-terminus antibody may be used as the immobilization antibody, and an anti-Aβ42 antibody may be used as the labeled antibody.

Further, in cases where amyloid β1-40 is to be measured by the sandwich method, an antibody (anti-Aβ40 antibody) that specifically binds to the C-terminal region of amyloid βx-40 and an antibody (anti-Aβ N-terminus antibody) that specifically binds to the N-terminal region including the first amino acid of amyloid β may be used. As described above, these antibodies may be either a commercially available antibody or an antibody produced by a well-known method. An anti-Aβ40 antibody may be used as the immobilization antibody, and an anti-Aβ N-terminus antibody may be used as the labeled antibody. Alternatively, an anti-Aβ N-terminus antibody may be used as the immobilization antibody, and an anti-Aβ40 antibody may be used as the labeled antibody.

The anionic polymer may be separately added to the antigen-antibody reaction system, or may be preliminarily added to a solution containing an antibody-immobilized solid phase such as antibody-immobilized particles, or to a reagent such as a sample diluent for diluting the blood sample. Alternatively, the anionic polymer may be preliminarily added to the blood sample.

The final concentration of the anionic polymer in the antigen-antibody reaction system is usually about 0.01 g/L to 50 g/L, about 0.02 g/L to 25 g/L, about 0.03 g/L to 10 g/L, about 0.04 g/L to 5 g/L, or preferably about 0.05 g/L to 2.5 g/L.

The immunoassay method *per se* is well known, and the present invention is applicable to any immunoassay method as described above. More specifically, as the immunoassay method of the present invention, a well-known immunoassay method may be used as it is except that the antigen-antibody reaction is carried out in the presence of the anionic polymer. Further, as described above, since immunoassays using CSF as a sample have already been carried out, kits employed for these immunoassays may be used as they are.

It is known that, similar to CSF, blood contains both Aβ₁₋₄₂ and Aβ₁₋₄₀. It is also known that, for both CSF and blood, diagnostic specificity can be increased by taking their ratio, that is, Aβ₁₋₄₂/Aβ₁₋₄₀. Thus, both Aβ₁₋₄₂ and Aβ₁₋₄₀ are quantified, and the ratio between these is calculated. In cases where the ratio is lower than those in healthy individuals, a patient is judged to be likely to have Alzheimer's disease. In general, the sensitivity of an immunoassay decreases when the length of time between the collection of the blood sample and the immunoassay is long. However, as concretely described in the following Examples, it was unexpectedly discovered that, in cases where the antigen-antibody reaction is carried out in the presence of an anionic polymer, the sensitivity hardly decreases (that is, Aβ₁₋₄₂/Aβ₁₋₄₀ hardly changes over time) even when the length of time between the collection of the blood sample and the immunoassay is long.

The kit of the present invention is not limited as long as it is a kit for carrying out the above-described immunoassay method of the present invention, and as long as it comprises at least an anti-amyloid β antibody and an anionic polymer. In cases where the immunoassay is a sandwich method, the kit also comprises a solid phase on which an anti-amyloid β antibody is immobilized. In cases where the solid phase is particles such as magnetic particles, the kit also comprises the particles (usually in the form of a particle liquid). By inclusion of an anionic polymer in a well-known amyloid β immunoassay kit, the kit of the present invention can be provided. As described above, the anionic polymer may be included in an anti-amyloid β antibody-immobilized particle liquid or in a reagent such as a sample diluent, or may be separately included in the kit.

### EXAMPLES

The present invention is described below concretely based on Examples. However, the present invention is not limited to the following Examples.

### <Preparation of Immobilization Particles>

In 10 mM MES buffer (pH 5.0), 0.2 mg/mL of a mouse anti-Aβ42 antibody that specifically binds to the C-terminal region of amyloid βx-42 was added to 0.01 g/mL of magnetic particles. The resulting mixture was incubated with gentle stirring at 25°C for 1 hour. After the reaction, the magnetic particles were magnetically collected using a magnet, and then the particles were washed with a washing liquid (50 mM Tris buffer, 150 mM NaCl, 2.0% BSA, pH 7.2), to obtain anti-Aβ42 antibody-immobilized particles. The anti-Aβ42 antibody-immobilized particles obtained were suspended in a particle diluent (50 mM Tris buffer, 1 mM EDTA 2Na, 0.1% NaN₃, 2.0% BSA, pH 7.2), to obtain an anti-Aβ42 antibody-immobilized particle solution.

Using a mouse anti-Aβ40 antibody that specifically binds to the C-terminal region of amyloid βx-40, anti-Aβ40 antibody-immobilized particles and an anti-Aβ40 antibody-immobilized particle solution were similarly prepared.

### <Preparation of Alkaline Phosphatase-Labeled Antibody>

Desalted alkaline phosphatase (ALP) was mixed with N-(4-maleimidobutyloxy)-succinimide (GMBS) (final concentration, 0.3 mg/mL), and the resulting mixture was left to stand at 30°C for 1 hour to carry out maleimide modification. Subsequently, in a coupling reaction liquid (100 mM phosphate buffer, 1 mM EDTA 2Na, pH 6.3), a mouse anti-Aβ N-terminus antibody that specifically binds to the N-terminal region including the first amino acid of amyloid β, which antibody was prepared as Fab', was mixed with the maleimide-modified ALP at a molar ratio of 1:1. Thereafter, the reaction was allowed to proceed at 25°C for 1 hour. Purification by column chromatography was carried out using Superdex 200 10/300 (manufactured by GE), to obtain an alkaline phosphatase-labeled antibody (ALP-labeled antibody). The ALP-labeled antibody was suspended in a labeled-body diluent (50 mM MES buffer, 150 mM NaCl, 0.3 mM ZnCl₂, 1 mM MgCl₂, 0.1% NaN₃, 2.0% BSA, pH 6.8), to obtain an ALP-labeled antibody solution.

### <Method of Measuring Amyloid β1-42 (Aβ₁₋₄₂)>

Into a reaction vessel, 50 µL of a sample and 50 µL of the anti-Aβ42 antibody-immobilized particle solution were dispensed, and the resulting mixture was stirred. Thereafter, the mixture was incubated at 37°C for 8 minutes, and then B/F separation using a magnetic field was carried out followed by washing. Into the reaction vessel, 50 µL of the ALP-labeled antibody solution was further dispensed. The resulting mixture was stirred, and then incubated at 37°C for 8 minutes, followed by B/F separation using a magnetic field, and washing. Thereafter, 200 µL of Lumipulse (registered trademark) substrate liquid (manufactured by Fujirebio Inc.), which contains a chemiluminescent substrate 3-(2'-spiroadamantan)-4-methoxy-4-(3"-phosphoryloxy)phenyl-1,2-dioxetane disodium salt (AMPPD), was dispensed into the reaction vessel. The resulting mixture was stirred, and then incubated at 37°C for 4 minutes, followed by measuring the amount of luminescence using a luminometer. The actual measurement was carried out using a fully automated chemiluminescent enzyme immunoassay system (Lumipulse L2400 (manufactured by Fujirebio Inc.)).

### <Method of Measuring Amyloid β1-40 (Aβ₁₋₄₀)>

Into a reaction vessel, 30 µL of a sample and 50 µL of the anti-Aβ40 antibody-immobilized particle solution were dispensed, and the resulting mixture was stirred. Thereafter, the mixture was incubated at 37°C for 8 minutes, and then B/F separation using a magnetic field was carried out followed by washing. Into the reaction vessel, 50 µL of the enzyme-labeled antibody solution was further dispensed. The resulting mixture was stirred, and then incubated at 37°C for 8 minutes, followed by B/F separation using a magnetic field, and washing. Thereafter, 200 µL of Lumipulse substrate liquid (manufactured by Fujirebio Inc.), which contains AMPPD, was dispensed into the reaction vessel. The resulting mixture was stirred, and then incubated at 37°C for 4 minutes, followed by measuring the amount of luminescence using a luminometer. The actual measurement was carried out using a fully automated chemiluminescent enzyme immunoassay system (Lumipulse L2400 (manufactured by Fujirebio Inc.)).

### Example 1

The effect of addition of dextran sulfate sodium in measurement of Aβ₁₋₄₂ and Aβ₁₋₄₀ using blood samples was studied.

To 4.5 mL each of blood samples (serum and EDTA-2K plasma) whose levels of Aβ₁₋₄₂ and Aβ₁₋₄₀ were below the measurement limit, 500 µL of a cerebrospinal fluid (CSF) containing the Aβ₁₋₄₂ antigen and the Aβ₁₋₄₀ antigen at concentrations of about 794 pg/mL and about 7744 pg/mL, respectively, was added to prepare antigen-containing sera (Serum 1 and Serum 2) and an antigen-containing plasma (EDTA 2K). In addition, the Aβ₁₋₄₂ antigen and the Aβ₁₋₄₀ antigen were added to Tris-buffered physiological saline (TBS buffer, pH 7.4) at the same concentrations as in the blood samples, to prepare an antigen-containing TBS buffer (Buffer). Using the prepared samples, both Aβ₁₋₄₂ and Aβ₁₋₄₀ were measured according to the methods described above in the "method of measuring Aβ₁₋₄₂" and the "method of measuring Aβ₁₋₄₀", respectively. As the dextran sulfate sodium, dextran sulfate sodium (mass average molecular weight, 5000 (hereinafter referred to as "molecular weight, 5000"; the same applies to others)) was added to the antibody-immobilized particle solution to 0.5 g/L, and the effect of the addition was studied. The final concentration of dextran sulfate sodium in the antigen-antibody reaction system was 0.25 g/L in the case of Aβ₁₋₄₂, and 0.31 g/L in the case of Aβ₁₋₄₀. In addition, as a blank, each sample (serum, EDTA-2K plasma, or TBS buffer) to which no antigen was added was similarly subjected to the measurement, to provide the obtained count as a blank value. Each count value was determined by subtracting the blank value from the count of each antigen-containing sample. The results for Aβ₁₋₄₂ are shown in Table 1, and the results for Aβ₁₋₄₀ are shown in Table 2.

**[Table 1]**

| Aβ1-42 | | Dextran sulfate sodium 5000 | |
|---|---|---|---|
| | | No addition | 0.5 g/L |
| Count value | Buffer | 5396 | 5488 |
| | Serum 1 | 4311 | 4860 |
| | Serum 2 | 3071 | 4533 |
| | EDTA 2K | 4434 | 5290 |
| Ratio to Buffer (%) | Buffer | 100.0 | 100.0 |
| | Serum 1 | 79.9 | 88.6 |
| | Serum 2 | 56.9 | 82.6 |
| | EDTA 2K | 82.2 | 96.4 |

**[Table 2]**

| Aβ1-40 | | Dextran sulfate sodium 5000 | |
|---|---|---|---|
| | | No addition | 0.5 g/L |
| Count value | Buffer | 360904 | 337955 |
| | Serum 1 | 338872 | 323636 |
| | Serum 2 | 371949 | 333830 |
| | EDTA 2K | 131505 | 309068 |
| Ratio to Buffer (%) | Buffer | 100.0 | 100.0 |
| | Serum 1 | 93.9 | 95.8 |
| | Serum 2 | 103.1 | 98.8 |
| | EDTA 2K | 36.4 | 91.5 |

As shown in Table 1, under the condition where dextran sulfate sodium was not added, the count values of Aβ₁₋₄₂ of the serum samples and the plasma sample were lower than the count value of the buffer sample. Relative to the count value in the buffer sample, which was taken as 100%, the count value in the serum sample Serum 2 was 56.9%, showing a large decrease.

On the other hand, in the case where dextran sulfate sodium was added to the antibody-immobilized particle solution before the measurement of Aβ₁₋₄₂, the serum samples and the plasma sample both showed increases in the count value, resulting in recovery of the count value to not less than 80% relative to the count value in the buffer sample.

Further, as shown in Table 2, under the condition where dextran sulfate sodium was not added, the count value of Aβ₁₋₄₀ in the plasma sample was lower than the count value in the buffer sample. Relative to the count value in the buffer sample, which was taken as 100%, the count value in the plasma sample EDTA 2K was 36.4%, showing a large decrease. On the other hand, in the case where dextran sulfate sodium was added to the antibody-immobilized particle solution before the measurement of Aβ₁₋₄₀, the count value in the plasma sample increased, resulting in recovery of the count value to not less than 90% relative to the count value in the buffer sample.

### Example 2

The concentration of dextran sulfate sodium added in the measurement of Aβ₁₋₄₂ in blood was studied. By the method described in Example 1, Aβ₁₋₄₂ was measured for the antigen-containing sera (Serum 1 and Serum 2), the antigen-containing plasma (EDTA 2K), and the antigen-containing TBS buffer (Buffer). As the dextran sulfate sodium, dextran sulfate sodium (mass average molecular weight, 5000) was added to the antibody-immobilized particle solution to achieve the concentration in Table 3 (0.1 g/L to 5.0 g/L), and the effect of the addition was studied. The final concentration of dextran sulfate sodium in the antigen-antibody reaction system was 0.05 g/L to 2.5 g/L. A blank value was obtained in the same manner as in Example 1 to calculate the count value. The results are shown in Table 3.

**[Table 3]**

| Aβ1-42 | | Dextran sulfate sodium 5000 | | | | | | |
|---|---|---|---|---|---|---|---|---|
| | | No addition | 0.1 g/L | 0.2 g/L | 0.5 g/L | 1 g/L | 2 g/L | 5 g/L |
| Count value | Buffer | 5396 | 5374 | 5452 | 5488 | 5301 | 5563 | 5743 |
| | Serum 1 | 4311 | 4494 | 4695 | 4860 | 4787 | 5055 | 5047 |
| | Serum 2 | 3071 | 4318 | 4387 | 4533 | 4633 | 4553 | 4574 |
| | EDTA 2K | 4434 | 5154 | 5499 | 5290 | 5547 | 5294 | 5655 |
| Ratio to Buffer (%) | Buffer | 100.0 | 100.0 | 100.0 | 100.0 | 100.0 | 100.0 | 100.0 |
| | Serum 1 | 79.9 | 83.6 | 86.1 | 88.6 | 90.3 | 90.9 | 87.9 |
| | Serum 2 | 56.9 | 80.3 | 80.5 | 82.6 | 87.4 | 81.8 | 79.6 |
| | EDTA 2K | 82.2 | 95.9 | 100.9 | 96.4 | 104.6 | 95.2 | 98.5 |

As shown in Table 3, in all study conditions of the dextran sulfate sodium concentration, the count value increased relative to the count value in the non-addition condition.

### Examples 3 to 5 and Comparative Examples 1 to 3

The effect of addition of various anionic polymers on the measurement of Aβ in a blood sample was studied. Measurement of Aβ₁₋₄₂ was carried out by the method described in Example 1 using the serum (antigen-containing serum, Serum 2) that showed especially remarkable decreases in the count value under the condition without addition of dextran sulfate sodium in Examples 1 and 2, and the antigen-containing TBS buffer (Buffer). As anionic polymers, dextran sulfate sodium (molecular weight, 5000) (Example 3), poly(sodium 4-styrenesulfonate) (molecular weight, 700,000) (Example 4), sodium dodecyl sulfate (SDS) (Comparative Example 1), sodium dodecylbenzene sulfonate (SDBS) (Comparative Example 2), sodium polyacrylate (molecular weight, 5100) (Example 5), and sodium N-lauroyl sarcosine (NLS) (Comparative Example 3) were used. Each of these anionic polymers was added to the antibody-immobilized particle solution to 0.5 g/L, and the effect of the addition was studied. Similar to Example 1, a blank value was obtained for the calculation of the count value. The results are shown in Table 4.

**[Table 4]**

| Aβ1-42 | | No addition | Dextran sulfate sodium | Poly(sodium 4-styrenesulfonate) | Sodium polyacrylate | SDS | SDBS | NLS |
|---|---|---|---|---|---|---|---|---|
| | Characteristic | - | Polymer | Polymer | Polymer | Surfactant | Surfactant | Surfactant |
| | Side chain | - | Sulfate group | Sulfo group | Carboxyl group | Sulfate group | Sulfo group | Carboxyl group |
| Count value | Buffer | 5396 | 5488 | 4474 | 6726 | 5232 | 5274 | 5128 |
| | Serum2 | 3071 | 4533 | 3462 | 4497 | 3025 | 3043 | 3096 |
| Ratio to | Buffer | 100.0 | 100.0 | 100.0 | 100.0 | 100.0 | 100.0 | 100.0 |
| Buffer (%) | Serum2 | 56.9 | 82.6 | 77.4 | 66.9 | 57.8 | 57.7 | 60.4 |

As shown in Table 4, under the condition where no anionic polymer was added, the count value in the serum sample (Serum 2) was 56.9% relative to the count value in the buffer sample. However, in the cases where the anionic polymer dextran sulfate sodium (Example 3), poly(sodium 4-styrenesulfonate) (Example 4), or sodium polyacrylate (Example 5) was added, the count value increased to 82.6%, 77.4%, or 66.9%, respectively. In particular, dextran sulfate sodium and poly(sodium 4-styrenesulfonate), which are anionic polymers comprising a side chain containing a sulfate group or a sulfo group, showed high recovery of the count value, so that they were shown to be especially effective. On the other hand, addition of SDS (Comparative Example 1), which is a surfactant comprising a side chain containing a sulfate group, SDBS (Comparative Example 2), which is a surfactant comprising a side chain containing a sulfo group, or NLS (Comparative Example 3), which comprises a side chain containing a carboxyl group, did not cause recovery of the count value.

Thus, addition of an anionic polymer to the reaction liquid was shown to be effective for the improvement of the decrease in the count value in the measurement of amyloid β in a blood sample. It was also shown that, among anionic polymers, anionic polymers comprising a side chain containing a sulfate group or a sulfo group are especially effective for the improvement.

### Example 6

The effect of the concentration of an anionic polymer added, in measurement of Aβ in a blood sample was studied. To 5 mL each of blood samples (serum and EDTA-2K plasma) whose levels of Aβ₁₋₄₂ are below the measurement limit, 50 µL of a synthetic Aβ₁₋₄₂ antigen at a concentration of 50,000 pg/mL was added to prepare antigen-containing sera (Serum 1 and Serum 2) and an antigen-containing plasma (EDTA 2K). In addition, the synthetic Aβ₁₋₄₂ antigen was added to Tris-buffered physiological saline (TBS buffer, pH 7.4) to achieve the same concentration as in the blood samples, to prepare an antigen-containing TBS buffer (Buffer). Using each sample prepared, Aβ₁₋₄₂ was measured by the method described in Example 1. As the anionic polymer, instead of dextran sulfate sodium, heparin sulfate sodium, which is an anionic polymer comprising a side chain containing a sulfate group, was added to the antibody-immobilized particle solution to achieve the concentration shown in Table 5 (0.5 g/L or 5.0 g/L), and the effect of the addition was studied. The final concentration of heparin sulfate sodium in the antigen-antibody reaction system was 0.25 g/L or 2.5 g/L. Similar to Example 1, a blank value was obtained for the calculation of the count value. The results are shown in Table 5.

**[Table 5]**

| Aβ1-42 | Additive | No addition | Heparin sulfate sodium | |
|---|---|---|---|---|
| | Concentration (g/L) | | 0.5 | 5 |
| Count value | Buffer | 24726 | 23413 | 22235 |
| | Serum 1 | 17121 | 18269 | 18226 |
| | Serum 2 | 15230 | 16667 | 16448 |
| | EDTA 2K | 17566 | 18295 | 18454 |
| Ratio to Buffer (%) | Buffer | 100.0 | 100.0 | 100.0 |
| | Serum 1 | 59.2 | 78.0 | 82.0 |
| | Serum 2 | 51.6 | 71.2 | 74.0 |
| | EDTA 2K | 71.0 | 78.1 | 83.0 |

As shown in Table 5, in all study conditions of the concentration of the heparin sulfate sodium added, the count value increased relative to the count value in the non-addition condition.

### Example 7

The effect of a difference in the molecular weight of the dextran sulfate sodium added, on the measurement of Aβ in a blood sample was studied. To 5 mL of the serum (Serum 2) that showed especially remarkable decreases in the count value under the condition without addition of dextran sulfate sodium in Examples 1 and 2, 50 µL of the synthetic Aβ₁₋₄₂ antigen at a concentration of 50,000 pg/mL was added to prepare an antigen-containing serum, and Aβ₁₋₄₂ was measured by the method described in Example 1. In addition, the synthetic Aβ₁₋₄₂ antigen was added to TBS buffer to achieve the same concentration as in the blood sample, to prepare an antigen-containing TBS buffer (Buffer), and measurement of Aβ₁₋₄₂ was similarly carried out. Two kinds of dextran sulfate having average molecular weights of 5000 and 50,000, respectively, were used. They were added to the antibody-immobilized particle solution to achieve a concentration of 7 g/L. The final concentration of dextran sulfate sodium in the antigen-antibody reaction system was 3.5 g/L. In addition, a blank value was obtained in the same manner as in Example 1 to calculate the count value. The results are shown in Table 6.

**[Table 6]**

| Aβ1-42 | Additive | No addition | Dextran sulfate sodium | |
|---|---|---|---|---|
| | Average molecular weight | | 5000 | 50000 |
| Count value | Buffer | 18610 | 19163 | 17952 |
| | Serum 2 | 12408 | 15501 | 16847 |
| Ratio to Buffer (%) | Buffer | 100.0 | 100.0 | 100.0 |
| | Serum 2 | 56.7 | 80.9 | 93.8 |

As shown in Table 6, under the condition where dextran sulfate sodium was not added, the count value in the serum sample (Serum 2) was 66.7% relative to the count value in the buffer sample. However, in the cases where dextran sulfate sodium (molecular weight, 5000) or dextran sulfate sodium (molecular weight, 50,000) was added, the count value increased to 80.9%, or 93.8%, respectively.

Thus, it was shown that addition of an anionic polymer such as dextran sulfate sodium having an average molecular weight of not less than 5000 to the reaction liquid is effective for improving the decrease in the count value in the measurement of amyloid β in a blood sample.

### Example 8

For the purpose of studying the reason why the count value was lower in the blood samples than in the buffer sample, EDTA-2K plasma and cerebrospinal fluid (CSF) were fractionated using a gel filtration column. The conditions were as follows. As an apparatus, AKTA FPLC (manufactured by GE Healthcare) was used. As a column, Superose 6 10×300 (manufactured by GE Healthcare) was used. The flow rate was 0.3 mL/minute. The buffer was D-PBS(-). The application volume was 1 mL. The liquid volume of each fraction was 1 mL. To each fraction obtained, the Aβ₁₋₄₂ antigen was added to 1000 pg/mL, to prepare a sample. Using each sample prepared, Aβ₁₋₄₂ was measured by the method described in Example 1. Dextran sulfate sodium (molecular weight, 5000) was added to the antibody-immobilized particle solution to 2.5 g/L, and the effect of the addition was studied. The final concentration of dextran sulfate sodium in the antigen-antibody reaction system was 1.3 g/L. As a control sample, TBS buffer to which Aβ₁₋₄₂ antigen was added at 1000 pg/mL was used to carry out the measurement in the same manner. The resulting ratio (%) of the count value in each sample to the count value in the control sample is shown in Table 7. Regarding the molecular weight, a molecular weight marker (manufactured by BioRad) was subjected to the operation under the above conditions to prepare an approximate expression for calculation of the molecular weight in each fraction.

**[Table 7]**

| | | | EDTA 2K plasma | | CSF | |
|---|---|---|---|---|---|---|
| Dextran sulfate sodium 5000 concentration | | | 0 g/L | 2.5 g/L | 0 g/L | 2.5 g/L |
| | Fraction No. | Molecular weight (kDa) | | | | |
| Count relative to Buffer (%) | 1 | 72957.1 | 98.1 | 102.5 | 96.2 | 95.9 |
| | 2 | 37374.1 | 105.1 | 106.2 | 103.2 | 103.7 |
| | 3 | 19145.8 | 127.7 | 116.5 | 100.4 | 95.6 |
| | 4 | 9807.9 | 115.6 | 117.0 | 101.2 | 98.3 |
| | 5 | 5024.4 | 97.3 | 112.4 | 100.4 | 94.3 |
| | 6 | 2573.9 | 101.6 | 126.9 | 99.9 | 98.1 |
| | 7 | 1318.5 | 97.3 | 120.4 | 100.8 | 98.4 |
| | 8 | 575.4 | 88.3 | 111.1 | 99.1 | 99.8 |
| | 9 | 346 | 51.0 | 106.2 | 98.7 | 98.2 |
| | 10 | 177.3 | 90.8 | 102.3 | 99.3 | 97.6 |
| | 11 | 90.8 | 104.8 | 97.5 | 100.2 | 97.4 |
| | 12 | 46.5 | 105.8 | 98.0 | 127.4 | 121.7 |
| | 13 | 23.8 | 108.3 | 101.7 | 226.0 | 216.9 |
| | 14 | 12.2 | 102.6 | 101.2 | 106.9 | 107.1 |
| | 15 | 5.3 | 101.6 | 104.4 | 99.4 | 99.8 |
| | 16 | 3.2 | 93.4 | 101.5 | 105.9 | 102.8 |
| | 17 | 1.6 | 98.0 | 99.2 | 98.7 | 99.0 |
| | 18 | 0.8 | 104.4 | 105.5 | 100.2 | 99.9 |
| | 19 | 3.4 | 103.9 | 104.3 | 101.2 | 98.0 |

As shown in Table 7, in the EDTA 2K plasma, the count value ratio decreased to 61% in the fraction of about 300 to 400 kDa under the condition where dextran sulfate sodium was not added. However, the addition of dextran sulfate sodium resulted in recovery of the count value to almost the same value (106%) as in the control sample. On the other hand, in the CSF, under the condition where dextran sulfate sodium was not added, none of the fractions showed a decrease in the count value relative to the value in the control sample, and there was no fraction showing a large difference in the count value ratio between the case where dextran sulfate sodium was added and the case where dextran sulfate sodium was not added.

It is thus thought that the component that decreases the count value is present only in the blood sample, and that the effect of the component can be avoided by the addition of the anionic polymer.

### Example 9

The effect of an anionic polymer on measurement of Aβ₁₋₄₂ and Aβ₁₋₄₀ in serum samples and EDTA 2Na plasma samples derived from healthy individuals was studied. The blood samples were prepared by collecting blood using Plain (10-mL blood collection tube, manufactured by Terumo Corporation) and EDTA 2Na (7-mL blood collection tube, manufactured by Terumo Corporation), leaving the collected blood to stand at room temperature for 30 minutes, carrying out centrifugation at 1200×g for 10 minutes, and then collecting the supernatant.

In order to evaluate the effect of storage of the samples, the samples were stored at 4°C for 0 day, 1 day, 2 days, or 3 days after the preparation, and then used to measure Aβ₁₋₄₂ and Aβ₁₋₄₀ according to the "method of measuring Aβ₁₋₄₂" and the "method of measuring Aβ₁₋₄₀". As the anionic polymer, dextran sulfate sodium (molecular weight, 5000) was added to the antibody-immobilized particle solution to 0.5 g/L, and the effect of the addition was studied. The measured values, and the ratio of each measured value to the value in the case of storage for 0 day under each condition are shown in Table 8. In the method of calculation of the measured values, each of the synthetic Aβ₁₋₄₂ peptide and the synthetic Aβ₁₋₄₀ peptide was used to prepare calibrators at 0 pg/mL, 10 pg/mL, 100 pg/mL, and 1000 pg/mL. Approximation expressions were prepared based on count values obtained by measurement of the calibrators, and the count values obtained from each sample were fitted to the approximation expressions to calculate measured values.

**[Table 8-1]**

| | | Aβ1-42 | | | | | | | |
|---|---|---|---|---|---|---|---|---|---|
| Dextran sulfate sodium 5000 | | 0 g/L | | | | 2.5 g/L | | | |
| Number of days of storage at 4°C | | 0 | 1 | 2 | 3 | 0 | 1 | 2 | 3 |
| Measured value | EDTA 2Na plasma #1 | 13.3 | 12.2 | 10.8 | 10.8 | 19.2 | 17.0 | 15.2 | 16.4 |
| | EDTA 2Na plasma #2 | 12.6 | 12.9 | 11.4 | 12.2 | 19.6 | 16.0 | 15.2 | 14.9 |
| | Serum #1 | 6.3 | 3.1 | 2.2 | 2.6 | 10.1 | 9.9 | 9.9 | 9.4 |
| | Serum #2 | 2.5 | 2.4 | 2.2 | 2.1 | 10.5 | 9.7 | 9.7 | 8.3 |
| Ratio to 0 day (%) | EDTA 2Na plasma #1 | 100.0 | 91.7 | 81.2 | 81.2 | 100.0 | 88.5 | 79.2 | 85.4 |
| | EDTA 2Na plasma #2 | 100.0 | 102.4 | 90.5 | 96.8 | 100.0 | 81.6 | 77.6 | 76.0 |
| | Serum #1 | 100.0 | 49.2 | 34.9 | 41.3 | 100.0 | 98.0 | 98.0 | 93.1 |
| | Serum #2 | 100.0 | 96.0 | 88.0 | 84.0 | 100.0 | 92.4 | 92.4 | 79.0 |

**[Table 8-2]**

| | | Aβ1-40 | | | | | | | |
|---|---|---|---|---|---|---|---|---|---|
| Dextran sulfate sodium 5000 | | 0 g/L | | | | 2.5 g/L | | | |
| Number of days of storage at 4°C | | 0 | 1 | 2 | 3 | 0 | 1 | 2 | 3 |
| Measured value | EDTA 2Na plasma #1 | 147.3 | 146.1 | 129.9 | 128.8 | 171.2 | 163.2 | 152.9 | 145.5 |
| | EDTA 2Na plasma #2 | 141.0 | 137.7 | 126.7 | 124.5 | 164.5 | 153.7 | 144.1 | 138.9 |
| | Serum #1 | 119.4 | 110.8 | 97.5 | 92.2 | 150.5 | 144.2 | 132.3 | 120.5 |
| | Serum #2 | 123.8 | 113.7 | 97.3 | 94.6 | 153.4 | 141.1 | 129.1 | 118.3 |
| Ratio to 0 day (%) | EDTA 2Na plasma #1 | 100.0 | 99.2 | 88.2 | 87.4 | 116.2 | 110.8 | 103.8 | 98.8 |
| | EDTA 2Na plasma #2 | 100.0 | 97.7 | 89.9 | 88.3 | 116.7 | 109.0 | 102.2 | 98.5 |
| | Serum #1 | 100.0 | 92.8 | 81.7 | 77.2 | 126.0 | 120.8 | 110.8 | 100.9 |
| | Serum #2 | 100.0 | 91.8 | 78.6 | 76.4 | 123.9 | 114.0 | 104.3 | 95.6 |

As shown in Table 8, in the cases where dextran sulfate sodium was added to the reagent, increases in the count value and the measured value were found for both the serum samples and the plasma samples. In the course of the measurement that was carried out over time, the measured value tended to decrease.

In measurement of amyloid β, the ratio between Aβ₁₋₄₂ and Aβ₁₋₄₀ is compared. In view of this, the ratio of Aβ₁₋₄₂ to Aβ₁₋₄₀ was calculated for each measurement condition in the present Example. The results are shown in Table 9.

**[Table 9]**

| | | Aβ1-42/1-40 | | | | Aβ1-42/1-40 | | | |
|---|---|---|---|---|---|---|---|---|---|
| Dextran sulfate sodium 5000 | | 0 g/L | | | | 2.5 g/L | | | |
| Number of days of storage at 4°C | | 0 | 1 | 2 | 3 | 0 | 1 | 2 | 3 |
| Measured value | EDTA 2Na plasma #1 | 0.090 | 0.084 | 0.083 | 0.084 | 0.112 | 0.104 | 0.099 | 0.113 |
| | EDTA 2Na plasma #2 | 0.089 | 0.094 | 0.090 | 0.098 | 0.119 | 0.104 | 0.105 | 0.107 |
| | Serum #1 | 0.053 | 0.028 | 0.023 | 0.028 | 0.067 | 0.069 | 0.075 | 0.078 |
| | Serum #2 | 0.020 | 0.021 | 0.023 | 0.022 | 0.068 | 0.069 | 0.075 | 0.070 |
| Ratio to 0 day (%) | EDTA 2Na plasma #1 | 100.0 | 92.5 | 92.1 | 92.9 | 100.0 | 92.9 | 88.6 | 100.5 |
| | EDTA 2Na plasma #2 | 100.0 | 104.8 | 100.7 | 109.7 | 100.0 | 87.4 | 88.5 | 90.0 |
| | Serum #1 | 100.0 | 53.0 | 42.8 | 53.4 | 100.0 | 102.3 | 111.5 | 116.2 |
| | Serum #2 | 100.0 | 104.5 | 112.0 | 109.9 | 100.0 | 100.4 | 109.8 | 102.5 |

As shown in Table 9, when the serum was subjected to the measurement using the reagent to which dextran sulfate sodium was not added, the value of the Aβ₁₋₄₂/Aβ₁₋₄₀ ratio was found to have changed over time. However, when the serum was subjected to the measurement using the reagent to which dextran sulfate sodium was added, the value of the Aβ₁₋₄₂/Aβ₁₋₄₀ ratio was found not to have changed over time, indicating that the Aβ₁₋₄₂/Aβ₁₋₄₀ ratio obtained is not affected by the storage period.

It was thus found that addition of an anionic polymer such as dextran sulfate sodium leads to not only increased count values of Aβ₁₋₄₂ and Aβ₁₋₄₀, but also an Aβ₁₋₄₂/Aβ₁₋₄₀ ratio not affected by the storage period.

### Example 10

Using the reagent to which dextran sulfate sodium was added, Aβ₁₋₄₂ and Aβ₁₋₄₀ in serum samples of patients with Alzheimer's disease (AD) and non-AD patients were measured.

Aβ₁₋₄₂ and Aβ₁₋₄₀ in serum samples of AD patients (26 cases) and non-AD patients (22 cases ) were measured by the following method. First, 50 µL of a sample and 250 µL of an antibody-immobilized particle solution to which dextran sulfate sodium (molecular weight, 5000) was added to 0.5 g/L were dispensed into a reaction vessel, and the resulting mixture was stirred. The final concentration of dextran sulfate sodium in the antigen-antibody reaction system was 0.42 g/L. Thereafter, the mixture was incubated at 37°C for 8 minutes, and then B/F separation using a magnetic field was carried out followed by washing. Into the reaction vessel, 250 µL of the enzyme-labeled antibody solution was further dispensed. The resulting mixture was stirred, and then incubated at 37°C for 8 minutes, followed by B/F separation using a magnetic field, and washing. Thereafter, 200 µL of Lumipulse (registered trademark) substrate liquid (manufactured by Fujirebio Inc.), which contains the chemiluminescent substrate AMPPD, was dispensed into the reaction vessel. The resulting mixture was stirred, and then incubated at 37°C for 4 minutes, followed by measuring the amount of luminescence using a luminometer. The actual measurement was carried out using a fully automated chemiluminescent enzyme immunoassay system (Lumipulse G1200 (manufactured by Fujirebio Inc.)). The results are shown in Fig. 1. Approximation expressions were prepared using the same calibrators as in Example 6, and measured values of Aβ₁₋₄₂ and Aβ₁₋₄₀ were calculated from the counts in each sample.

Fig. 1 is a box plot of the Aβ₁₋₄₂/Aβ₁₋₄₀ ratios calculated from the measured values obtained. As a result of the measurement using the reagent containing dextran sulfate sodium, the Aβ₁₋₄₂/Aβ₁₋₄₀ ratios in the AD patients were found to be significantly lower than the Aβ₁₋₄₂/Aβ₁₋₄₀ ratios in the non-AD patient group (p-value, 0.0022 (Wilcoxon-Mann-Whitney test)), indicating that the addition of an anionic polymer to the reaction liquid is useful also in the measurement of Aβ using a patient sample.

It was thus shown that a reagent to which an anionic polymer such as dextran sulfate sodium is added is useful for the diagnosis of Alzheimer's disease.

## Claims

1. A method of immunoassay of amyloid β in a blood sample, the method comprising carrying out the immunoassay in the presence of an anionic polymer.

2. The method according to claim 1, wherein the anionic polymer is an anionic polymer comprising a side chain containing a sulfate group or a sulfo group.

3. The method according to claim 2, wherein the anionic polymer is at least one selected from the group consisting of dextran sulfate and salts thereof; polystyrene sulfonic acid and salts thereof; and heparin sulfate and salts thereof.

4. The method according to claim 3, wherein the anionic polymer is dextran sulfate sodium.

5. The method according to any one of claims 1 to 4, wherein the immunoassay is a sandwich method, the method comprising bringing, in the presence of the anionic polymer, the amyloid β in the blood sample into contact with an anti-amyloid β antibody or an antigen-binding fragment thereof immobilized on a solid phase, to measure the amyloid β.

6. The method according to any one of claims 1 to 5, wherein the amyloid β is amyloid β1-40 or amyloid β1-42.

7. A kit for immunoassay of amyloid β in a blood sample, the kit comprising: an anti-amyloid β antibody or an antigen-binding fragment thereof; and an anionic polymer.

8. The kit according to claim 7, wherein the immunoassay is a sandwich method, the kit comprising: a solid phase on which an anti-amyloid β antibody or an antigen-binding fragment thereof is immobilized; and an anionic polymer.

9. The kit according to claim 8, wherein the solid phase is particles, the kit comprising a particle liquid containing the particles and the anionic polymer.

## Patentansprüche

1. Amyloid-β-Immunassay-Verfahren an einer Blutprobe, wobei das Verfahren das Ausführen des Immunassays in Gegenwart eines anionischen Polymers umfasst.

2. Verfahren nach Anspruch 1, wobei das anionische Polymer ein anionisches Polymer ist, das eine Seitenkette umfasst, die eine Sulfatgruppe oder eine Sulfogruppe enthält.

3. Verfahren nach Anspruch 2, wobei das anionische Polymer zumindest ein aus der aus Dextransulfat und Salzen davon; Polystyrolsulfonsäure und Salzen davon; und Heparinsulfat und Salzen davon bestehenden Gruppe ausgewähltes ist.

4. Verfahren nach Anspruch 3, wobei das anionische Polymer Dextransulfat-Natrium ist.

5. Verfahren nach einem der Ansprüche 1 bis 4, wobei der Immunassay ein Sandwich-Verfahren ist, wobei das Verfahren das In-Kontakt-Bringen des Amyloids β in der Blutprobe mit einem Anti-Amyloid-β-Antikörper oder einem antigenbindenden Fragment davon, der/das auf einer festen Phase immobilisiert ist, in Gegenwart des anionischen Polymers umfasst, um das Amyloid β zu messen.

6. Verfahren nach einem der Ansprüche 1 bis 5, wobei das Amyloid β Amyloid β1-40 oder Amyloid β1-42 ist.

7. Set für einen Amyloid-β-Immunassay an einer Blutprobe, wobei das Set Folgendes umfasst: einen Anti-Amyloid-β-Antikörper oder ein antigenbindendes Fragment davon; und ein anionisches Polymer.

8. Set nach Anspruch 7, wobei der Immunassay ein Sandwich-Verfahren ist und das Set Folgendes umfasst: eine feste Phase, auf der ein Anti-Amyloid-β-Antikörper oder ein antigenbindendes Fragment davon immobilisiert ist; und ein anionisches Polymer.

9. Set nach Anspruch 8, wobei die feste Phase aus Partikeln besteht und das Set eine Partikelflüssigkeit umfasst, welche die Partikel und das anionische Polymer enthält.

## Revendications

1. Procédé de dosage immunologique d'amyloïde β dans un échantillon de sang, le procédé comprenant la réalisation du dosage immunologique en présence d'un polymère anionique.

2. Procédé selon la revendication 1, dans lequel le polymère anionique est un polymère anionique comprenant une chaîne latérale contenant un groupe sulfate ou un groupe sulfo.

3. Procédé selon la revendication 2, dans lequel le polymère anionique est au moins un polymère choisi dans le groupe constitué du sulfate de dextrane et de ses sels ; de l'acide polystyrène sulfonique et de ses sels ; et du sulfate d'héparine et de ses sels.

4. Procédé selon la revendication 3, dans lequel le polymère anionique est le sulfate de dextrane sodique.

5. Procédé selon l'une quelconque des revendications 1 à 4, dans lequel le dosage immunologique est un procédé sandwich, le procédé comprenant la mise en contact, en présence du polymère anionique, d'amyloïde β dans l'échantillon de sang avec un anticorps anti-amyloïde β ou un fragment de liaison à un antigène de celui-ci immobilisé sur une phase solide, pour mesurer le β-amyloïde.

6. Procédé selon l'une quelconque des revendications 1 à 5, dans lequel l'amyloïde β est l'amyloïde β1-40 ou l'amyloïde β1-42.

7. Kit pour dosage immunologique d'amyloïde β dans un échantillon de sang, le kit comprenant : un anticorps anti-amyloïde β ou un fragment de liaison à un antigène de celui-ci ; et un polymère anionique.

8. Kit selon la revendication 7, dans lequel le dosage immunologique est un procédé sandwich, le kit comprenant : une phase solide sur laquelle un anticorps anti-amyloïde β ou un fragment de liaison à un antigène de celui-ci est immobilisé ; et un polymère anionique.

9. Kit selon la revendication 8, dans lequel la phase solide est constituée de particules, le kit comprenant un liquide particulaire contenant les particules et le polymère anionique.
